# FASCICULE DE BREVET EUROPEEN

(11) **EP 4 202 417 B1**
(45) Date de publication et mention de la délivrance du brevet: **12.06.2024**
(21) Numéro de dépôt: 22216676.1
(22) Date de dépôt: 26.12.2022
(51) Int. Cl.: G01N 21/64

(54) **DISPOSITIF DE DÉTECTION BIOMOLÉCULAIRE PAR NANOPHOTODÉTECTEURS**
VORRICHTUNG ZUR BIOMOLEKULAREN DETEKTION MITTELS NANOFOTODETEKTOREN
BIOMOLECULAR DETECTION DEVICE USING NANOPHOTODETECTORS

(30) Priorité: 27.12.2021 FR 2114563
(43) Date de publication de la demande: 28.06.2023
(73) Titulaire: Commissariat à l'énergie atomique et aux énergies alternatives, 75015 Paris (FR); Centre National de la Recherche Scientifique, 75016 Paris Cedex 16 (FR)
(72) Inventeur: CONSTANTIN, Olivier, 38054 GRENOBLE cedex 09 (FR); HOCEVAR, Moira, 38000 GRENOBLE (FR); JAFFAL, Ali, 38054 GRENOBLE cedex 09 (FR); MAILLEY, Pascal, 38054 GRENOBLE cedex 09 (FR); MONROY, Eva, 38054 GRENOBLE cedex 09 (FR)
(74) Mandataire: INNOV-GROUP

(56) Documents cités:
- US-A1- 2012 113 419
- MARIA SPIES ET AL: "Nanowire photodetectors based on wurtzite semiconductor heterostructures", ARXIV.ORG, CORNELL UNIVERSITY LIBRARY, 201 OLIN LIBRARY CORNELL UNIVERSITY ITHACA, NY 14853, 27 avril 2019 (2019-04-27), XP081268008, DOI: 10.1088/1361-6641/AB0CB8

## Description

### DOMAINE TECHNIQUE

Le domaine technique de l'invention est la détection de biomolécules par transduction optique effectuée par des nanofils.

### ART ANTERIEUR

Les dispositifs permettant de détecter des biomolécules peuvent être basés sur une détection optique. Les biomolécules à détecter sont préalablement marquées à l'aide d'un marqueur luminescent, de type fluorophore ou boite quantique (quantum dot). La détection est effectuée par un photodétecteur matriciel, de type imageur CCD, CMOS ou matrices de photodétecteurs à avalanches (APD : Avalanche Photodetectors). Cependant, les photodétecteurs matriciels usuels présentent une matrice de détection dont le pas spatial est généralement supérieur à 1 µm. Par pas spatial, on entend la distance entre deux pixels adjacents. Un tel pas spatial est considéré comme trop élevé pour obtenir un dispositif permettant d'adresser un grand nombre de détections en parallèle. Ces dispositifs présentent également une sensibilité limitée, qui semble insuffisante pour effectuer une détection de séquences après un faible nombre d'amplifications.

Certains biocapteurs sont basés sur une détection de charges portées par les biomolécules cibles à détecter. Il s'agit par exemple de transistors à effet de champ. Ce type de dispositif permet d'effectuer une détection rapide, avec une bonne sensibilité. De plus, la technologie CMOS (semi-conducteur à oxyde métallique complémentaire) permet une fabrication de dispositifs comportant un grand nombre de capteurs. Il est ainsi possible d'effectuer un grand nombre d'analyses en parallèle. Cependant, ce type de capteur peut être sensible à des paramètres environnementaux affectant l'échantillon, par exemple pH ou température ou interactions avec des biomolécules non ciblées ou d'ions induisant des erreurs de détection. Le recours à ce type de biocapteurs dans des applications de séquençage d'ADN (acide désoxyribonucléique) suppose une certaine redondance de mesures, de façon à augmenter la robustesse des mesures. Les séquenceurs ADN basés sur des nanopores présentent les mêmes inconvénients.

Des capteurs photoniques basés sur des nanofils ont été développés à des fins de détection de fragments d'ADN. C'est par exemple le cas de la publication Singh et al « Silicon nanowire optical rectangular waveguide biosensor for DNA Hybridization », IEEE Photonics Technology Letters, 2018, 30(12) 1123-1126. Dans cette publication, on détecte une hybridation d'ADN en détectant une variation d'indice de réfraction lors de l'hybridation

La publication Irrera. "New génération of ultrasensitive label-free optical Si nanowire-based biosensors", ACS Photonics 2018,5,471-479 décrit un biocapteur à base de nanofils utilisés pour détecter la CRP (C-réactive protéin) dans du sérum humain.

La publication US 2012/113419 A1 divulgue un dispositif pour détecter une molécule d'intérêt dans un échantillon par spectroscopie Raman exaltée de surface, le dispositif comprenant un photodétecteur et une pluralité de nanofils photoémetteurs arrangés sur un même substrat.

La publication Spies M. et Monroy E., "Nanowire photodetectors based on wurtzite semiconductor heterostructures", Cornell University Library, 2019, DOI: 10.1088/1361-6641/AB0CB8, discute des photodétecteurs à base de nanofils.

Les inventeurs ont conçu un dispositif d'analyse d'un échantillon compact et sensible, permettant de tirer profit des capacités de détection optique de nanofils. Le dispositif peut permettre une analyse simultanée ou séquentielle de différentes biomolécules.

### EXPOSE DE L'INVENTION

Un premier objet de l'invention est un dispositif de détection d'au moins une biomolécule d'intérêt dans un échantillon, le dispositif comportant :
- un substrat, comportant au moins une première électrode;
- une structure multicouches, comportant au moins une deuxième électrode;
- des nanofils, s'étendant entre la première électrode et la deuxième électrode, parallèlement à un axe transversal;
- une couche d'encapsulation s'étendant autour des nanofils, entre le substrat et la structure multicouches, la couche d'encapsulation étant formée d'un matériau isolant ;
- la structure multicouche comportant :
   - une couche conductrice, formant chaque deuxième électrode;
   - une couche d'interface, électriquement isolante, recouvrant chaque deuxième électrode, chaque deuxième électrode étant interposée entre la couche d'interface et un nanofil, la couche d'interface étant délimitée par une surface de fonctionnalisation, la couche d'interface étant configurée pour être disposée entre l'échantillon et la deuxième électrode, de telle sorte que la surface de fonctionnalisation forme une interface entre le dispositif et l'échantillon ;
- la structure multicouche étant telle que
   - la surface de fonctionnalisation est configurée pour capturer sélectivement la biomolécule d'intérêt;
   - la deuxième électrode et la couche d'interface sont transparentes dans une bande spectrale de détection ;
le dispositif étant tel que :
- chaque nanofil comporte une jonction de type homojonction, ou une hétérojonction, ou une jonction Schottky entre la première électrode et la deuxième électrode ;
- la première électrode et la deuxième électrode sont configurées pour être raccordées à un circuit de détection;
- de telle sorte que chaque nanofil forme un nanophotodétecteur d'une lumière émise au niveau de la surface de fonctionnalisation, la lumière détectée par chaque nanofil induisant un signal électrique de détection dans le circuit de détection.

Ainsi, la couche d'interface permet une isolation des nanofils vis-à-vis de l'échantillon.

Chaque nanofil peut comporter :
- une homojonction de type pn ;
- ou une hétérojonction ;
- ou une jonction Schottky de type p-métal ou n-métal.

Au moins un nanofil peut comporter une première partie et une deuxième partie, séparées par la jonction, la première partie étant formée d'un semi-conducteur dopé n, la deuxième partie étant formée d'un semi-conducteur dopé p, la jonction formant une homojonction ou une hétérojonction.

Au moins un nanofil peut comporter une première partie et une deuxième partie, séparées par la jonction, la première partie étant formée d'un semi-conducteur, la deuxième partie, adjacente d'une électrode, étant formée d'un métal, la jonction formant une jonction Schottky ;. Selon un mode de réalisation, la première partie et la deuxième partie s'étendent, selon l'axe transversal, de part et d'autre de la jonction.

Selon un mode de réalisation, la deuxième partie entoure la première partie, autour de l'axe transversal.

Selon un mode de réalisation, au moins un nanofil comporte un puits quantique ou un point quantique au niveau de la jonction.

Selon un mode de réalisation, la surface de fonctionnalisation est segmentée en différentes surfaces élémentaires, chaque surface élémentaire formant un site de capture de la biomolécule d'intérêt.

Selon un mode de réalisation,
- la couche d'interface comporte deux sous-couches, empilées l'une sur l'autre, formant une sous-couche inférieure et une sous-couche supérieure, la sous-couche inférieure étant interposée entre la couche conductrice et la sous-couche supérieure ;
- la sous-couche supérieure comporte des puits, débouchant dans la sous-couche inférieure, chaque puits étant disposé face à un nanofil, chaque puits formant une partie de la surface de fonctionnalisation ;
- la surface de fonctionnalisation est segmentée au niveau de chaque puits, de façon que chaque puits forme un site de capture de la biomolécule d'intérêt.

Entre les puits, la sous-couche supérieure peut ne pas être fonctionnalisée, ou comporter un revêtement évitant un dépôt d'une biomolécule d'intérêt.

Selon un mode de réalisation, le dispositif comporte plusieurs nanofils, s'étendant entre une même première électrode et une même deuxième électrode, les nanofils formant une grappe de nanofils. Le dispositif peut comporter plusieurs grappes de nanofils, distantes les unes des autres, de telle sorte qu'un nanofil d'une grappe est plus proche d'un autre nanofil de ladite grappe que d'un autre nanofil d'une autre grappe, les nanofils d'une même grappe s'étendant entre une même première électrode et une même deuxième électrode.

Selon un mode de réalisation, dit de multiplexage, le dispositif comporte plusieurs nanofils, le dispositif étant tel que :
- plusieurs premières électrodes sont formées sur le substrat, et plusieurs deuxièmes électrodes sont formées sur la structure multicouche, chaque nanofil s'étendant entre une première électrode et une deuxième électrode ;
- chaque première électrode est reliée à une première unité d'adressage, configurée pour sélectionner au moins une première électrode ;
- chaque deuxième électrode est reliée à une deuxième unité d'adressage, configurée pour sélectionner au moins une deuxième électrode ;
- de telle sorte que le circuit de détection détecte un signal de détection induit par chaque nanofil s'étendant entre la première électrode et la deuxième électrode sélectionnées.

La première unité d'adressage peut être configurée pour sélectionner successivement plusieurs premières électrodes. La deuxième unité d'adressage peut être configurée pour sélectionner successivement plusieurs deuxièmes électrodes.

Selon une possibilité,
- le substrat et la structure multicouche s'étendent parallèlement à un axe longitudinal et un axe latéral, l'axe latéral et l'axe longitudinal étant sécants l'un de l'autre, dans un plan sécant de l'axe transversal;
- chaque première électrode est disposée parallèlement à l'axe longitudinal ;
- chaque deuxième électrode est disposée parallèlement à l'axe latéral.

Avantageusement,
- la surface de fonctionnalisation est fonctionnalisée par une sonde biologique de détection, la sonde biologique de détection étant configurée pour capturer sélectivement une biomolécule d'intérêt ;
- de telle sorte qu'une capture de la biomolécule d'intérêt entraîne une variation de la lumière de fluorescence détectée par au moins un nanofil lorsque la surface de fonctionnalisation est éclairée par une lumière d'excitation d'un marqueur fluorescent greffé sur la biomolécule d'intérêt ou sur la sonde biologique de détection.

La sonde biologique de détection peut être une balise moléculaire.

Selon une possibilité,
- la surface de fonctionnalisation est segmentée en plusieurs portions;
- chaque portion de la surface de fonctionnalisation s'étend face à au moins un nanofil ;
- différentes portions de la surface de fonctionnalisation sont respectivement fonctionnalisées par des sondes biologiques de détection configurées pour capturer sélectivement différentes biomolécules d'intérêt.

Un autre objet de l'invention est un procédé de détection d'au moins une biomolécule d'intérêt à l'aide d'un dispositif selon le premier objet de l'invention, la surface de fonctionnalisation étant préalablement fonctionnalisée avec une sonde biologique de détection configurée pour capturer sélectivement la biomolécule d'intérêt, le dispositif comportant :
a) disposition d'un échantillon, susceptible de contenir la biomolécule d'intérêt, au contact de la surface de fonctionnalisation ;
b) connexion du circuit de détection aux bornes d'une première électrode et d'une deuxième électrode ;
c) exposition de la surface de fonctionnalisation à une lumière d'excitation, dans une bande spectrale d'excitation d'un marqueur fluorescent, le marqueur fluorescent étant greffé sur la sonde biologique de détection ou sur la biomolécule d'intérêt, le marqueur fluorescent étant susceptible d'émettre une lumière de fluorescence, dans la bande spectrale de détection, lorsqu'il est illuminé par la lumière d'excitation ;
d) en fonction du signal de détection, détection d'une variation d'une lumière de fluorescence détectée par au moins un nanofil, la variation traduisant une capture de la biomolécule d'intérêt par une sonde biologique de détection.

Selon un mode de réalisation,
- le dispositif est un dispositif selon le mode de réalisation de type multiplexage décrit en lien avec le premier objet de l'invention ;
- les étapes b) à d) sont réitérées, en modifiant, entre deux itérations successives, la première électrode sélectionnée ou la deuxième électrode sélectionnée.

L'invention sera mieux comprise à la lecture de l'exposé des exemples de réalisation présentés, dans la suite de la description, en lien avec les figures listées ci-dessous.

### FIGURES

Les figures 1A à 1D montrent les principaux composants d'un dispositif selon l'invention.
Les figures 2A à 2E schématisent des étapes de fabrication de nanofils selon un procédé dit ascendant.
Les figures 3A à 3C représentent différents agencements possibles des nanofils.
La figure 4A et la figure 4B schématisent des étapes de fabrication de nanofils selon un autre procédé, dit descendant.
La figure 5 représente un exemple de dispositif selon l'invention, dans lequel les électrodes sont réparties selon un agencement matriciel.
Les figures 6A à 6E schématisent différentes possibilités de couplage entre une sonde biologique de détection et une biomolécule cible, de façon que la capture de la biomolécule cible induise une émission ou une extinction d'une lumière de fluorescence.
Les figures 7A et 7B schématisent une balise moléculaire avant et après capture de la biomolécule cible.
Les figures 8A et 8B montrent deux structures différentes de nanofils.

### EXPOSE DE MODES DE REALISATION PARTICULIERS

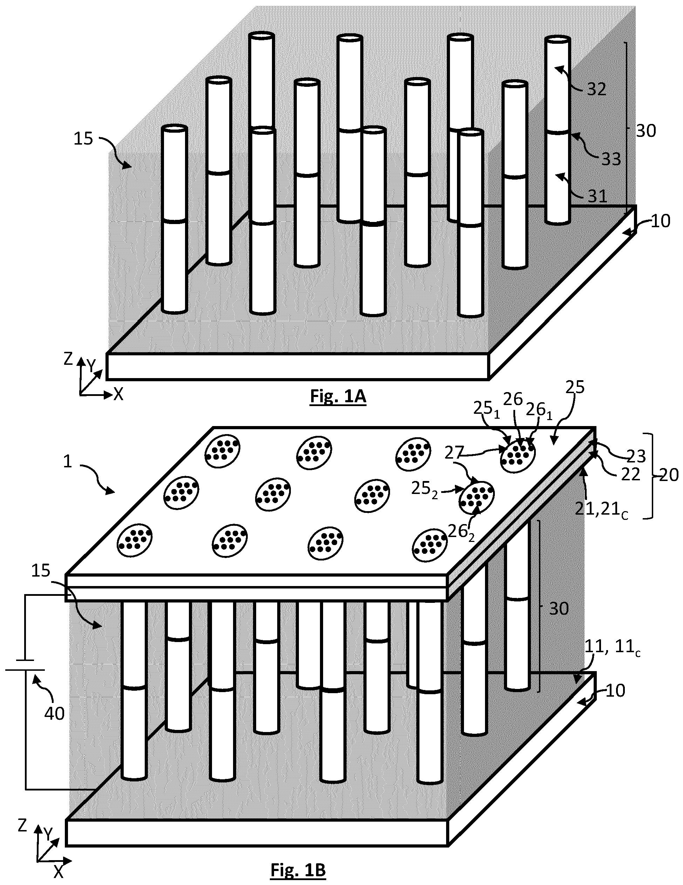

On a représenté, sur les figures 1A à 1C, un premier exemple de dispositif d'analyse 1 permettant une mise en oeuvre de l'invention. Le dispositif d'analyse 1 est configuré pour être placé au contact d'un échantillon 2, comportant par exemple un milieu liquide susceptible de contenir une biomolécule d'intérêt 3. La biomolécule d'intérêt 3 peut être choisie parmi : une protéine, par exemple une enzyme, un fragment de protéine, un anticorps, un antigène, un monobrin de nucléotides, une hormone.

Le dispositif comporte un substrat 10, formant ou comportant au moins une première électrode 11_{c}. Dans l'exemple représenté sur la figure 1A, le substrat est un substrat de silicium cristallin, par exemple un substrat Si d'orientation cristalline (111). Le substrat 10 est délimité par une surface, dite première surface 11, comportant une première électrode 11_{c}. Dans l'exemple de la figure 1A, la première surface 11 est formée de Si comportant des régions conductrices. Selon une autre possibilité le substrat 10 fait l'objet d'un dépôt d'une couche conductrice, par exemple du graphène, formant tout ou partie de la première surface 11.

Des nanofils 30 sont formés sur le substrat 10, et plus précisément à partir de la première surface 11. La première surface 11 s'étend selon un plan P_{XY}. Le plan P_{XY} est défini par un axe longitudinal X et un axe latéral Y. Les axes X et Y sont sécants, et de préférence perpendiculaires l'un par rapport à l'autre. Les nanofils s'étendent parallèlement à un axe transversal Z sécant du plan Dans les modes de réalisation décrits par la suite, l'axe transversal est perpendiculaire du plan P_{XY}. La première surface 11 est conductrice au moins au niveau de l'intersection avec chaque nanofil 30. La totalité de la première surface 11 peut être conductrice.

Selon d'autres configurations, les nanofils peuvent être inclinés et non pependiculaires au plan P_{XY}. Par exemple, si l'orientation cristalline du matériau formant le substrat 10 est (001), les nanofils peuvent croître selon une direction (111), donc en biais par rapport au plan P_{XY}.

Les nanofils 30 ont de préférence un diamètre compris entre 1 nm et 500 nm et une hauteur, selon l'axe transversal Z, comprise entre 300 et 1000 nm, voire 10000 nm.

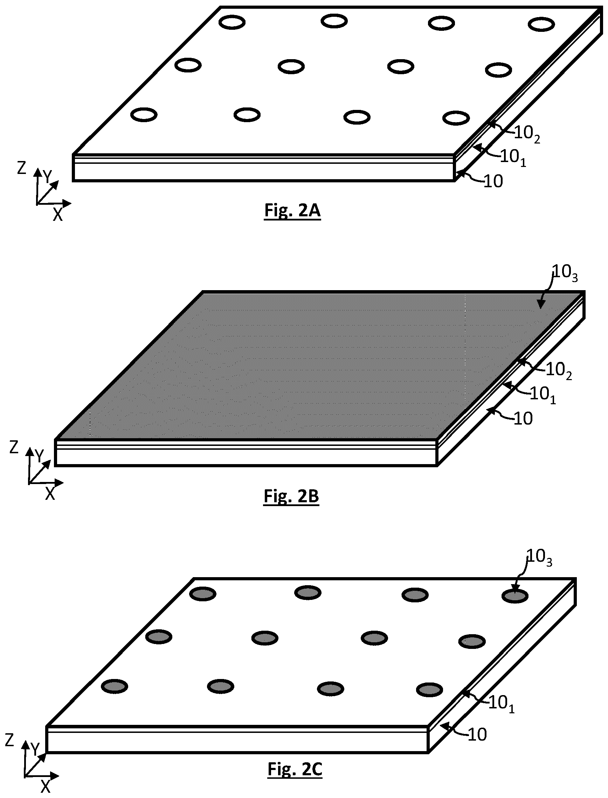

Les nanofils 30 peuvent être synthétisés directement sur le substrat 10, comme décrit en lien avec les figures 2A à 2E. Les nanofils peuvent être formés sur un autre substrat, puis transférés sur le substrat 10. Le transfert peut être effectué comme décrit dans la publication Valente J. et al « Light-Emitting GaAs Nanowires on a Flexible Substrate », Nano Lett 2018 18 7 4206-4213.

Les nanofils 30 s'étendent, à partir de la première surface 11, jusqu'à une deuxième surface 21 délimitant une structure multicouches 20. De même que la première surface 11, la deuxième surface 21 est conductrice au moins au niveau de l'intersection avec chaque nanofil 30. Dans l'exemple représenté sur la figure 1B, la deuxième surface 21 est formée à partir d'une couche 22 d'un matériau conducteur transparent dans une bande spectrale de détection décrite ci-après. Il peut par exemple s'agir d'ITO (oxyde d'indium étain).

Chaque nanofil est formé d'un ou plusieurs matériaux semi-conducteurs, et éventuellement d'un matériau métallique. Chaque nanofil comporte une jonction 33. Dans l'exemple représenté, la jonction 33 est une homojonction : chaque nanofil comporte une première partie 31, adjacente de la première surface 11, et une deuxième partie 32, adjacente de la deuxième surface 21. Les première et deuxième parties sont formées d'un même matériau semi-conducteur, avec respectivement deux dopages différents : ainsi, la première partie 31 et la deuxième partie 32 sont respectivement formés d'un même matériau semi-conducteur respectivement dopé n et p ou p et n. Dans l'exemple représenté, la première partie 31 est formée de GaAs (arséniure de gallium) dopé p et la deuxième partie est formée de GaAs dopé n. L'interface entre les deux parties forme la jonction pn 33.

De façon alternative, la jonction 33 peut être effectuée niveau d'un métal disposé sur ou au contact de la surface 21 de façon à établir une jonction semi-conducteur - métal de type Schottky.

Chaque nanofil comporte au moins un semi-conducteur choisi parmi les matériaux des colonnes III et V, usuellement désignés par le terme matériaux III-V, par exemple, GaAs. Il peut de préférence s'agir d'un matériau de la colonne III et d'arsenic, par exemple InAs (arséniure d'indium). Dans l'exemple représenté, les nanofils 30 sont formés de GaAs. D'autres matériaux semi-conducteurs peuvent être envisagés, par exemple, et de façon non limitative, Si, InGaAs, AlGaAs, InGaP, InGaN, GaN, ZnSe, ZnS, ZnO, ZnCdO.

Entre la première surface 11 et la deuxième surface 21, les nanofils 30 sont noyés dans une couche d'encapsulation 15 formée d'un matériau isolant. La couche d'encapsulation 15 peut être formée d'un matériau de type PMMA (Polyméthacrylate de méthyle), BCB (Benzocyclobuthène), SiO₂. La couche d'encapsulation 15 peut être déposée par spin-coating (dépôt à la tournette).

La couche d'encapsulation 15 est de préférence formée suite à la croissance des nanofils, préalablement au dépôt d'une deuxième conductrice 22, délimitée par la deuxième surface 21, et destinée à former des deuxièmes électrodes 21_{c}. Les deuxièmes électrodes 21_{c} sont formées, au niveau de la deuxième surface 21, à partir de la couche conductrice 22. La couche conductrice 22 peut être structurée de façon qu'au niveau de la deuxième surface 21, plusieurs deuxièmes électrodes 21_{c} soient électriquement isolées les unes des autres. Ainsi, la couche conductrice peut comporter des ouvertures ou des matériaux isolant délimitant les électrodes 21_{c}.

Outre la couche conductrice 22, la structure multicouches 20 comporte une couche d'interface 23, adjacente de la couche conductrice 22. La couche d'interface 23 est transparente. La couche d'interface 23 peut par exemple être formée d'une couche d'un polymère, par exemple le PMMA (Polyméthylméthacrylate). La couche d'interface 23 est isolante, en particulier au niveau de l'interface avec la couche conductrice 22. La couche d'interface 23 est de préférence plane. La couche d'interface 23 est destinée à former une interface entre la couche conductrice 22, formant les électrodes 21_{c}, et l'échantillon 2. Ainsi, la couche d'interface 23 s'étend entre la couche conductrice 22 et l'échantillon 2 à analyser. Un aspect important du dispositif est que l'échantillon n'est pas en contact direct avec les nanofils. Il est isolé de ces derniers par la couche d'interface 23.

La couche d'interface 23 est par exemple une couche mince formée de SiO₂ ou de PMMA. La surface de la couche d'interface 23, destinée à être en contact avec l'échantillon est une surface, dite surface de fonctionnalisation 25, fonctionnalisée par des sondes biologiques de détection 26. Il est important que la couche d'interface 23 soit formée d'un matériau présentant une auto-fluorescence aussi faible que possible dans la bande spectrale de détection.

La surface de fonctionnalisation 25, qui forme une interface entre la structure multicouches 20 et l'échantillon 2 à analyser, est destinée à être fonctionnalisée par des sondes biologiques de détection 26. Par sonde biologique de détection, on entend une espèce configurée pour capturer sélectivement une biomolécule d'intérêt. La sonde biologique 26 de détection peut être formée d'acides nucléiques, ou de protéines, ou d'anticorps, ou d'antigènes, ou d'enzymes. La capture sélective se fait par couplage de la biomolécule d'intérêt sur la sonde de détection, par des interactions spécifiques : hybridation (ADN, ARN), repliement (aptamère), interactions chimiques multiples faible énergie (anticorps, enzyme), adsorption (protéine).

La couche d'interface 23 peut être nanostructurée, de façon que des nanopuits 27 soient formés à l'interface entre la structure multicouches 20 et l'échantillon à analyser. Le diamètre ou la plus grande diagonale des nanopuits peut être comprise entre 70 nm et 700 nm. Les nanopuits 27 peuvent par exemple être agencés de façon matricielle, ou, de façon plus générale, selon un motif prédéterminé. La surface de fonctionnalisation est alors fonctionnalisée au niveau de chaque nanopuits 27, les espaces entre chaque puits n'étant pas fonctionnalisés. La formation des nanopuits peut être obtenue par amincissement local de la couche d'interface 23.

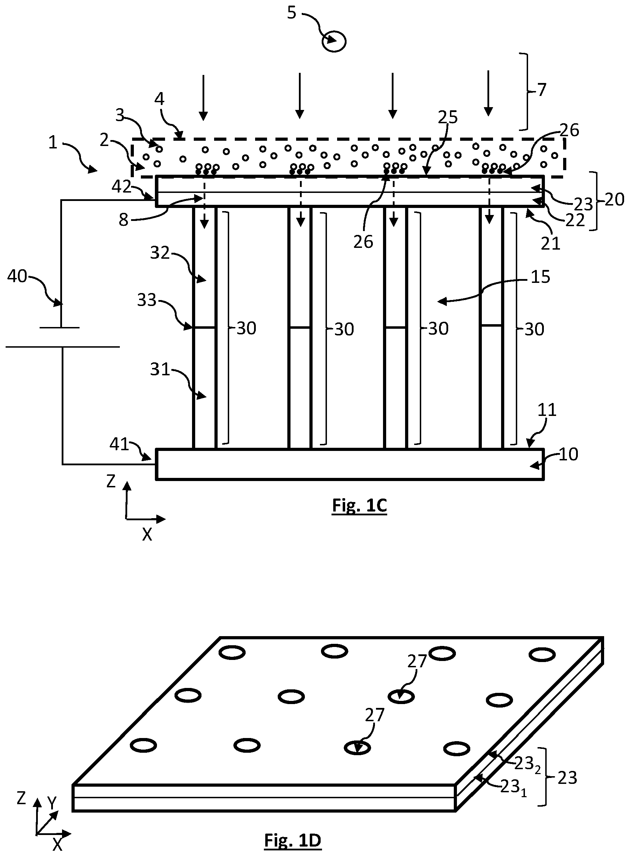

Selon une possibilité, représentée sur la figure 1D, la couche d'interface 23 comporte deux sous-couches superposées 23₁ et 23₂. La couche d'interface comporte une sous-couche inférieure 23₁ interposée entre la couche conductrice 22 et une sous-couche supérieure 23₂. Les nanopuits sont formés par amincissement local de la sous-couche supérieure 23₂, de façon que les nanopuits débouchent dans la sous-couche inférieure 23₁. La sous-couche supérieure 23₂ peut être formée d'un matériau non fonctionnalisable, par exemple un matériau anti-biofouling (anti-encrassement biologique). Une telle structuration permet que la fonctionnalisation de la surface de fonctionnalisation 25 soit effectuée uniquement au niveau des nanopuits 27, sur la sous-couche inférieure 23₁. Chaque nanopuits 27 forme ainsi un site de capture de l'espèce biologique d'intérêt 3.

De façon plus générale, la surface de fonctionnalisation 25 peut être fonctionnalisée selon un motif de fonctionnalisation prédéterminé. En dehors du motif de fonctionnalisation, la surface de fonctionnalisation n'est pas fonctionnalisée vis-à-vis de l'espèce biologique que l'on souhaite capturer.

La fonctionnalisation peut être effectuée par un traitement de la surface de fonctionnalisation 25, par exemple un traitement de surface plasma/oxygène. Lorsque la couche d'interface 23 est formée de PMMA, un traitement plasma/oxygène permet de former des fonctions carboxyles. Les sondes biologiques de détection peuvent être greffées sur la surface de fonctionnalisation par liaison covalente. A cette fin, les sondes biologiques comportent une fonction, par exemple une fonction amine, de façon à former une liaison covalente avec les fonctions de la surface de fonctionnalisation 25.

Lorsque la fonctionnalisation de la surface de fonctionnalisation 25 est effectuée selon un motif spatial, les parties de la surface de fonctionnalisation en dehors du motif spatial peuvent être revêtues d'un revêtement anti-biofouling (anti-encrassement biologique) de façon à éviter une adsorption non spécifique des biomolécules d'intérêt (ou cibles). Un exemple de matériau anti-biofouling est un polyéthylène glycol.

Le dépôt de sondes biologiques de détection peut être effectué par couplage chimique sur la surface de fonctionnalisation 25.

Selon une possibilité, différentes parties 25₁, 25₂ de la surface de fonctionnalisation 25 sont fonctionnalisées avec différentes sondes biologiques de détection 26₁, 26₂. Chaque partie de la surface de fonctionnalisation 25 est ainsi fonctionnalisée de façon à capturer sélectivement une biomolécule d'intérêt différente d'une autre partie de la surface de fonctionnalisation. De préférence, une même partie de la surface est fonctionnalisée avec une même sonde biologique de détection destinée à capturer une même biomolécule d'intérêt. Par exemple, lorsque les sondes biologiques de détection sont formées de fragments monobrins d'ADN, une même partie de la surface de fonctionnalisation est fonctionnalisée avec le même fragment d'ADN. Deux parties différentes sont fonctionnalisées avec des fragments d'ADN différents. Préalablement à la fonctionnalisation, les fragments d'ADN formant les sondes biologiques de détection sont préparés en utilisant des méthodes de l'art antérieur, par exemple une préparation d'une librairie de fragments d'ADN courts (<150 pb - pb = paires de bases) à partir d'un brin long extrait de cellules biologiques. Ainsi, le dispositif peut adresser simultanément ou séquentiellement différentes espèces biologiques d'intérêt.

Lorsque différentes parties de la surface de fonctionnalisation sont fonctionnalisées avec différentes sondes de détection, le dispositif permet d'effectuer une analyse dit multiplexée : l'analyse peut adresser simultanément ou successivement différentes biomolécules d'intérêt, par exemple différentes séquences de nucléotides.

Comme précédemment indiqué, chaque sonde biologique de détection 26 est configurée pour capturer une biomolécule d'intérêt 3. Par capture, on entend l'établissement d'une liaison entre la sonde de détection et la biomolécule d'intérêt, entraînant un couplage de la biomolécule d'intérêt sur la sonde de détection. La liaison entre la sonde de détection et la biomolécule d'intérêt peut être une liaison chimique (liaison dative, liaison hydrogène, pont disulfure), ou une liaison de type antigène-anticorps, ou une liaison physique (interactions électrostatiques, hydrophobes ou de Van der Walls) ou une hybridation lorsque la sonde de détection est une séquence de nucléotides et que la biomolécule d'intérêt est une séquence de nucléotides complémentaire.

Le dispositif 1 est basé sur une détection optique d'une capture d'une biomolécule d'intérêt par une sonde biologique de détection 26, induisant une réponse électrique du dispositif. Le dispositif comporte un circuit de détection 40, dont une première borne 41 est raccordée à une première électrode 11_{c}, sur le substrat 10, et une deuxième borne 42 est raccordée à une deuxième électrode 21_{c}, sur la structure multicouches 20. Le circuit de détection 40 permet de mesurer la différence de potentiel, ou un courant électrique, entre la première électrode 11_{c} et la deuxième électrode 21_{c}.

Sur la figure 1C, on a représenté une vue en coupe du dispositif. Un échantillon 2 liquide est disposé dans une chambre fluidique 4, au contact de la couche d'interface 23. L'échantillon est isolé des nanofils 30 par la couche d'interface 23. Ainsi, les nanofils ne sont pas en contact avec les biomolécules d'intérêt.

Lorsque le dispositif est en fonctionnement, une source de lumière 5 illumine la surface de fonctionnalisation 25, dans une bande spectrale d'excitation centrée sur une longueur d'onde d'excitation de fluorescence. Dans l'exemple représenté sur la figure 1C, la source de lumière génère une lumière d'excitation 7, qui se propage à travers l'échantillon 2 jusqu'à la surface de fonctionnalisation 25. La biomolécule d'intérêt 3 a préalablement fait l'objet d'un marquage par un marqueur fluorescent 6. Sous l'effet de l'illumination à la longueur d'onde d'excitation 7, le marqueur fluorescent 6 génère une lumière de fluorescence 8 dans une longueur d'onde de fluorescence, plus élevée que la longueur d'onde d'excitation. Le marqueur fluorescent 6 peut être un fluorophore ou une boite quantique.

D'une façon générale, la capture de la biomolécule d'intérêt entraîne une variation d'une émission d'une lumière de fluorescence 8, cette variation de fluorescence entraînant une variation de la réponse électrique du dispositif.

Dans l'exemple représenté sur la figure 1C, la capture de la biomolécule 3 par une des sondes de détection 26 entraîne une concentration de marqueurs fluorescents 6 sur la surface de fonctionnalisation 25, à l'aplomb des nanofils. Sous l'effet de la lumière d'excitation 7, des photons de fluorescence sont émis, formant la lumière de fluorescence 8. Du fait de la transparence de la couche d'interface 23, ou du matériau 22 constituant l'électrode 21_{c}, certains photons de fluorescence se propagent à travers un nanofil 30. Lorsqu'un photon de fluorescence est absorbé au niveau de la jonction 33, des paires électrons/trous sont formées. Sous l'effet de la différence de potentiel entre la première partie 31 et la deuxième partie 32, les électrons se propagent à travers la portion dopée n, vers le potentiel le plus élevé tandis que les trous se propagent en sens inverse à travers la portion dopée p. Il en résulte une augmentation du courant électrique circulant dans le circuit de détection 40.

Selon une autre possibilité, la capture de la biomolécule induit une réduction ou une extinction d'une émission des photons de fluorescence, par exemple par effet de quenching. En l'absence de capture, les photons de fluorescence sont émis, ce qui entraîne une circulation d'un courant électrique dans le circuit de détection 40. Suite à une capture, l'émission des photons de fluorescence est réduite, ou stoppée, ce qui entraîne une variation du courant électrique.

On comprend que le choix du matériau semi-conducteur formant le nanofil 30 dépend de la bande spectrale d'absorption dudit matériau, cette dernière devant à la fois contenir la longueur d'onde de fluorescence, et, de préférence, ne pas contenir la longueur d'onde d'excitation du marqueur fluorescent. Le nanofil agit ainsi en tant que nanophotodétecteur de la lumière de fluorescence, tout en n'étant pas sensible à la longueur d'onde d'excitation. Chaque nanofil forme ainsi un filtre vis-à-vis de la longueur d'onde d'excitation. Lorsque le matériau semi-conducteur est GaAs, le marqueur fluorescent peut être Cy3 (Cyanine) : longueur d'onde d'excitation 540nm et longueur d'onde d'émission comprise entre 555 et 600nm.

De façon alternative, lorsque le nanofil est sensible à la longueur d'onde d'excitation, la détection de la lumière de fluorescence peut être temporellement séparée de la détection de la longueur d'onde d'excitation.

Le diamètre des nanofils est contrôlé de façon à conférer une sensibilité dans une bande spectrale étroite. Par bande spectrale étroite, on entend une largeur de bande typiquement de quelques dizaines de nm, et de préférence inférieure à 100 nm. La largeur de bande correspond à la largeur à mi-hauteur du pic d'absorption sur le spectre d'absorption. La largeur de bande de détection est par exemple de l'ordre de 50 nm. Il est ainsi possible d'ajuster la bande spectrale de détection, de façon qu'elle comprenne la longueur d'onde de fluorescence du marqueur fluorescent et qu'elle ne comprenne pas la longueur d'onde d'excitation du marqueur fluorescent. La faculté de nanofils à former un photodétecteur sélectif en longueur d'onde a été décrite dans Mokkapati.S. et al « Optical design of nanowire absorbers for wavelenght sélective photodetectors », Sci. Rep. 5, 15339.

La sensibilité spectrale de détection peut être ajustée par l'incorporation de puits quantiques ou de boites quantiques (quantum dots) au niveau de la jonction 33. Cela permet d'obtenir une jonction 33 dont la composition est différente par rapport au reste du nanofil. La longueur d'onde détectée correspond alors à la longueur d'onde du gap défini par le puits ou la boîte quantique.

Chaque nanofil forme ainsi un nanophotodétecteur. L'invention est basée sur une combinaison d'une capture sélective et spatialisée d'une biomolécule cible et d'une détection de la capture par des nanophotodétecteurs. En utilisant différentes sondes de détection, adressant sélectivement différentes biomolécules cibles, réparties sur différentes zones spatiales de la surface de fonctionnalisation, l'invention permet une détection simultanée ou successive de la présence de différentes biomolécules.

On va à présent décrire différents aspects de conception du dispositif selon l'invention.

### Formation des nanofils

Les figures 2A à 2E schématisent des étapes de formation de nanofils à partir d'un substrat 10 de silicium. Les figures 2A à 2E correspondent à une approche dite montante (ou bottom-up). Le substrat 10 est formé de Si, d'orientation (111) et comporte une couche superficielle 10₁ de SiO₂, destinée à former une couche barrière, d'épaisseur 10nm - 15 nm. La couche de SiO₂ est recouverte d'une couche 10₂ de PMMA d'épaisseur 45 nm. Les couches 10₁ et 10₂ font l'objet d'une gravure, de façon à former des nanopuits isolés les uns des autres, selon un motif prédéterminé. Cf. figure 2A. Les nanopuits débouchent sur le substrat de Si. Une fine couche de métal 10₃, par exemple de l'or, d'épaisseur 5 à 10 nm est déposée sur la couche de SiO₂. Le métal ajouté, en l'occurrence l'or, joue le rôle de catalyseur. Cf. figure 2B. L'excès d'or entre les nanopuits est éliminé par lift of (retrait) de la couche 10₂ de PMMA. Cf. figure 2C. On obtient ainsi des ilots d'or 10₃ isolés les uns des autres, aux positions correspondant à la position des nanopuits préalablement formés.

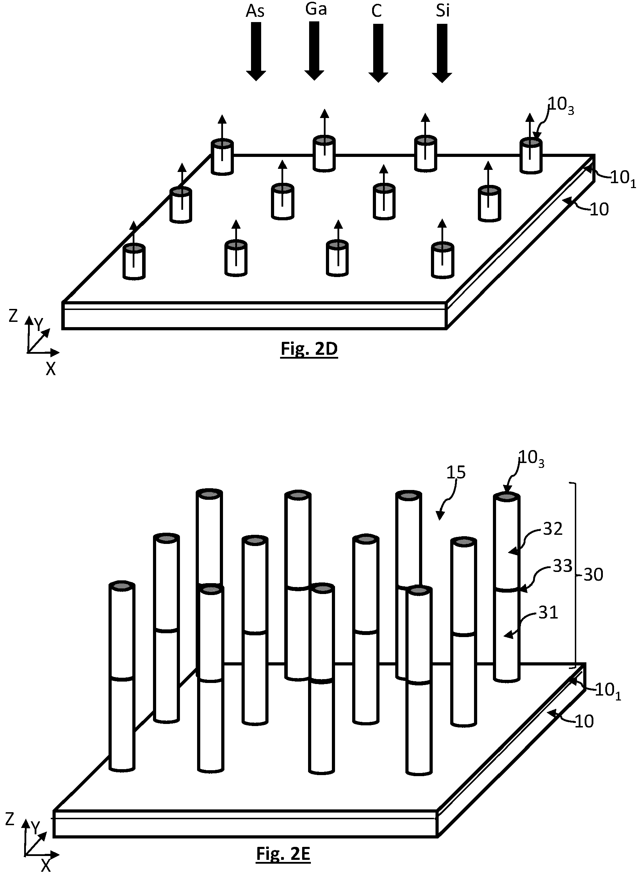

Après chauffage à une température supérieure à 450°C, les ilots forment des gouttes. Les gouttes d'or jouent le rôle de catalyseur. Les nanofils de semi-conducteur sont ensuite formés par épitaxie par jet moléculaire (MBE : molecular beam epitaxy). Cela consiste à envoyer un ou plusieurs jets moléculaires vers le substrat pour réaliser une croissance épitaxiale. Cf. figure 2D. Les jets moléculaire en phase vapeur comportent les espèces chimiques composant le nanofil semi-conducteur ainsi que les espèces dopantes. Les espèces moléculaires heurtent et diffusent dans les gouttelettes d'or. Lorsque ces dernières atteignent une saturation, la nucléation des nanofils se produit tout d'abord aux interfaces gouttelettes/substrat, puis aux interfaces gouttelettes/nanofils en formation.

Le procédé n'est pas limité à l'utilisation d'or en tant que catalyseur. D'autres catalyseurs peuvent être utilisés, par exemple Ga. Lorsque le catalyseur est Ga et que Ga constitue également un élément constituant le semi-conducteur, on parle de croissance de nanofils autocatalysée.

Ce procédé permet par exemple une croissance de nanofils en utilisant As, Ga ainsi que C et Si respectivement comme dopant p et n, la température de mise en oeuvre étant de 610°C. Le procédé se poursuit jusqu'à ce que les nanofils atteignent une hauteur prédéterminée. Cf. figure 2E.

Suite à l'étape représentée sur la figure 2E, la couche d'encapsulation 15 est formée entre les nanofils, par exemple par spin-coating. La couche d'encapsulation permet d'isoler électriquement les nanofils les uns des autres, et confère une meilleure tenue mécanique à l'ensemble. Une gravure plasma et/ou un pollissage peut être effectuée au niveau de l'extrémité des nanofils opposée au substrat, de façon à éliminer les résidus du catalyseur et homogénéiser la hauteur des nanofils et de la couche d'encapsulation 15.

La couche conductrice 22, puis la couche d'interface 23, sont ensuite successivement déposées sur l'ensemble formé par les nanofils et la couche d'encapsulation 15.

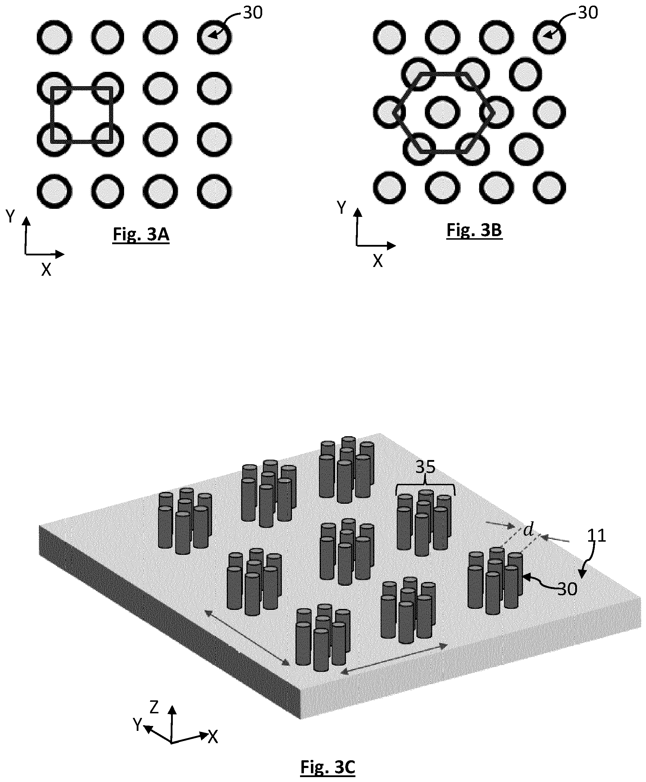

Dans le mode de réalisation décrit sur les figures 2A à 2E, la formation des nanopuits sur le substrat 10 permet de maîtriser la position des nanofils. Ainsi, les nanofils peuvent être agencés régulièrement, comme représenté sur les figures 3A et 3B. Sur les figures 3A et 3B, on a représenté des positions de nanofils sur le substrat 10 selon des motifs de maille respectivement carrée ou hexagonale. Le pas entre deux nanofils adjacents peut être faible, de l'ordre de deux fois le diamètre, ou être plus élevé, par exemple quelques à plusieurs centaines de nm.

Un autre avantage de l'approche montante, décrite en lien avec les figures 2A à 2E est qu'elle permet un contrôle plus précis de la structure cristalline des nanofils. L'approche montante permet une incorporation maîtrisée de boites quantiques (quantum dots) ou de puits quantiques, en contrôlant leur position, en particulier selon l'axe Z.

La figure 3C illustre une configuration selon laquelle les nanofils sont agencés en formant des grappes 35, ou clusters. Les nanofils d'un même cluster sont rapprochés les uns des autres, la distance d entre deux nanofils adjacents d'une même grappe étant de préférence supérieure ou égale au diamètre des nanofils. La distance entre deux grappes adjacentes peut être égale ou supérieure à deux fois la distance d. Chaque grappe peut présenter un arrangement régulier, comme représenté sur les figures 3A et 3B.

L'agencement des nanofils en clusters 35 peut être combiné avec une structuration de la surface de fonctionnalisation en nanopuits 27, comme décrit en lien avec la figure 1B. Dans ce cas, chaque nanopuits 27 s'étend face aux nanofils appartenant à un même cluster 35.

La disposition des nanofils selon un agencement prédéfini sur le substrat 10 n'est pas nécessaire. Selon une possibilité, des gouttes de catalyseur métallique sont réparties de façon aléatoire sur le substrat 10. Suite à l'adjonction, en phase vapeur, des molécules constituant le nanofil, par exemple Ga, As et des dopants, par exemple C, Si, les nanofils se développent à partir des positions initialement occupées, sur le substrat, par les gouttes de catalyseur.

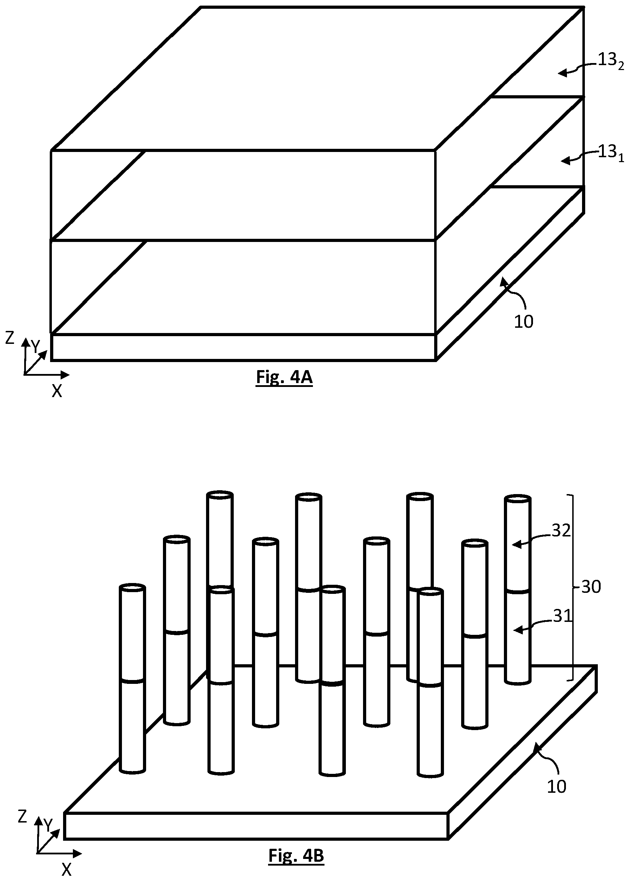

Selon une autre possibilité, les nanofils sont obtenus par gravure, selon une approche dite descendante (ou top down). Selon une telle possibilité, on forme, sur le substrat 10, une première couche 13₁ d'un semi-conducteur d'un premier dopage, par exemple dopage n, puis une deuxième couche 13₂, d'un semi-conducteur d'un autre dopage, par exemple dopage p. Cf. figure 4A. Les nanofils sont formés par gravure de la première couche 13₁ et de la deuxième couche 13₂. Cf. figure 4B. Une couche d'encapsulation 15 est ensuite formée autour des nanofils, comme précédemment décrit. La couche conductrice 22 est ensuite déposé sur l'extrémité libre des nanofils 30, pour former les électrodes 21c. La couche d'interface 23 est formée sur la couche conductrice 22. Un tel mode de fabrication peut aussi permettre la formation de nanofils comportant une jonction métal - semi-conducteur : la première couche 13₁ ou la deuxième couche 13₂ est formée d'un métal, tandis que l'autre couche est formée d'un semi-conducteur.

L'approche descendante permet d'obtenir des nanofils dont la composition est homogène en termes de dimensions et de profil de dopage. Cela est favorable à une intégration dans des dispositifs à grande échelle.

### Adressage électrique

Comme décrit en lien avec les figures 1A à 1C, chaque nanofil s'étend entre une première extrémité, sur une première surface 11 du substrat 10 et une deuxième extrémité, sur une deuxième surface 21 de la structure multicouche 20. La première surface 11 et la deuxième surface 21 sont conductrices au moins au niveau de chaque intersection avec un nanofil. Ainsi, au niveau de chaque intersection avec un nanofil, la première surface comporte une première électrode 11_{c} et la deuxième surface comporte une deuxième électrode 21_{c}. Dans l'exemple représenté sur les figures 1A à 1C, la première surface 11 et la deuxième surface 21 sont formées d'un matériau conducteur. Elles sont conductrices selon toute leur aire.

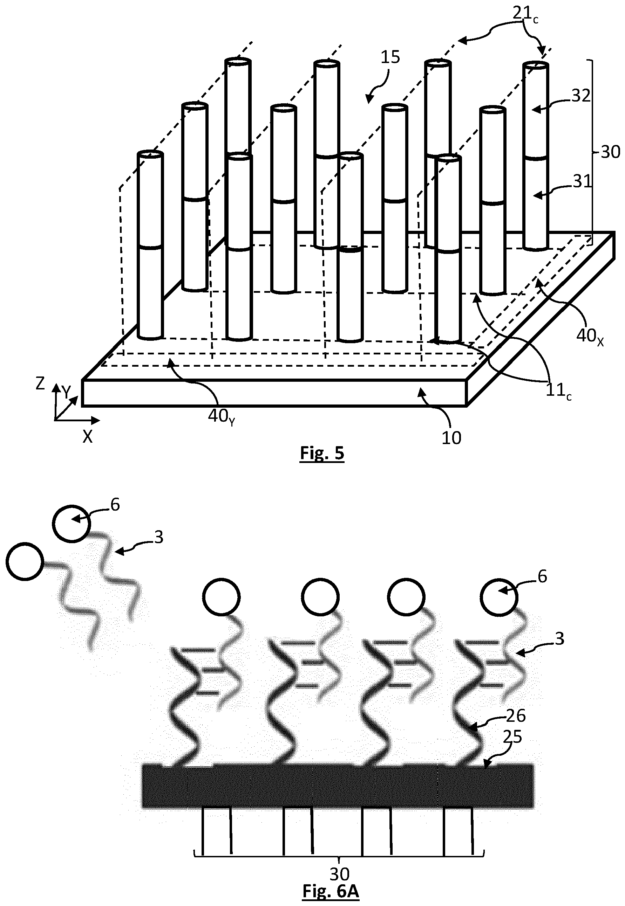

Les première et deuxième surfaces peuvent être structurées, et comporter différentes électrodes 11_{c}, 21_{c} isolées les unes des autres. Sur la figure 5, chaque électrode est matérialisée par une ligne en pointillés. Sur la première surface 11, chaque première électrode 11_{c} décrit une ligne, parallèle à l'axe longitudinal X. Sur la deuxième surface 21, chaque deuxième électrode 21_{c} décrit une colonne, parallèle à l'axe latéral Y. Chaque nanofil est fonctionnel lorsque les électrodes 11_{c}, 21_{c}, entre lesquelles il s'étend, sont polarisées. La structuration des électrodes en lignes / colonnes permet de sélectionner les nanofils fonctionnels, ces derniers s'étendant entre deux électrodes polarisées : selon cet agencement, les nanofils reliés à une ligne et à une colonne polarisées sont fonctionnels. Par nanofil fonctionnel, on entend un nanofil polarisé pour effectuer une détection de photons de fluorescence.

Plusieurs lignes et/ou plusieurs colonnes peuvent être polarisées simultanément ou successivement. Le circuit de détection 40 comporte :
- une unité d'adressage 40_{X}, destinée à polariser tout ou partie des premières électrodes 11_{c}, parallèles à l'axe X,
- et une unité d'adressage 40_{Y} destinée à polariser tout ou partie des deuxièmes électrodes 21_{c} parallèles à l'axe Y.

Lorsque les nanofils sont répartis en clusters, comme décrit en lien avec la figure 3C, les nanofils d'un même cluster sont de préférence raccordés à une même électrode, à la fois sur le substrat 10 et sur la structure multicouche 20. Les nanofils d'un même cluster sont ainsi simultanément fonctionnels. Les nanofils non reliés au circuit de détection ne sont pas fonctionnels.

### Fonctionnalisation - fluorescence

La fonctionnalisation de la surface de fonctionnalisation 25 est assurée par le greffage de sondes biologiques de détection 26 sur la surface de fonctionnalisation 25. Les sondes biologiques de détection 26 sont destinées à capturer sélectivement une biomolécule d'intérêt 3. Selon un mode de réalisation, en multiplexage, différentes sondes biologiques de détection 26, adressant respectivement différentes biomolécules d'intérêt, sont séparées sur la surface de fonctionnalisation 25. En référence avec la figure 3C, la partie de la surface de fonctionnalisation située en vis-à-vis d'un même cluster 35 peut être fonctionnalisée par une même sonde biologique de détection 26, de façon à adresser une même biomolécule d'intérêt 3. On tire alors profit de la détection potentielle, par plusieurs nanofils adjacents, d'une même biomolécule d'intérêt.

Deux parties différentes 25₁ 25₂ de la surface de fonctionnalisation 25, respectivement disposées en vis-à-vis de deux clusters différents, peuvent être fonctionnalisées par deux sondes biologiques de détection différentes, de façon à adresser deux biomolécules d'intérêt différentes. Une telle configuration convient particulièrement à la détection de différentes séquences de nucléotides cibles. La sonde biologique de détection comporte alors une séquence de nucléotides complémentaire de la séquence de nucléotides cible. Du fait de la sensibilité élevée de chaque nanofil, le nombre de nanofils composant une même grappe peut être relativement faible. Ainsi, la surface, dans le plan P_{XY}, de chaque grappe, est faible, chaque grappe adressant sélectivement une séquence de nucléotides cible différente d'une autre grappe. Il est ainsi possible de disposer un grand nombre de grappes, adressant respectivement différentes séquences de nucléotides cibles, dans un même dispositif compact.

Selon une possibilité, représentée sur la figure 6A, chaque biomolécule d'intérêt 3 fait l'objet d'un marquage par un marqueur fluorescent 6, comme précédemment décrit. Dans ce cas, l'accumulation de biomolécules d'intérêt 3 capturées au voisinage de la surface de fonctionnalisation 25 engendre une lumière de fluorescence détectable par les nanofils 30 les plus proches. La capture des biomolécules d'intérêt entraîne une augmentation de la lumière de fluorescence, qui est perceptible par le circuit de détection 40. Sur la figure 6A, la biomolécule d'intérêt 3 est une séquence d'ADN complémentaire d'un brin d'ADN formant la sonde biologique de détection 26. La capture de la biomolécule d'intérêt est obtenue par hybridation du brin complémentaire sur le brin formant la sonde biologique de détection 26.

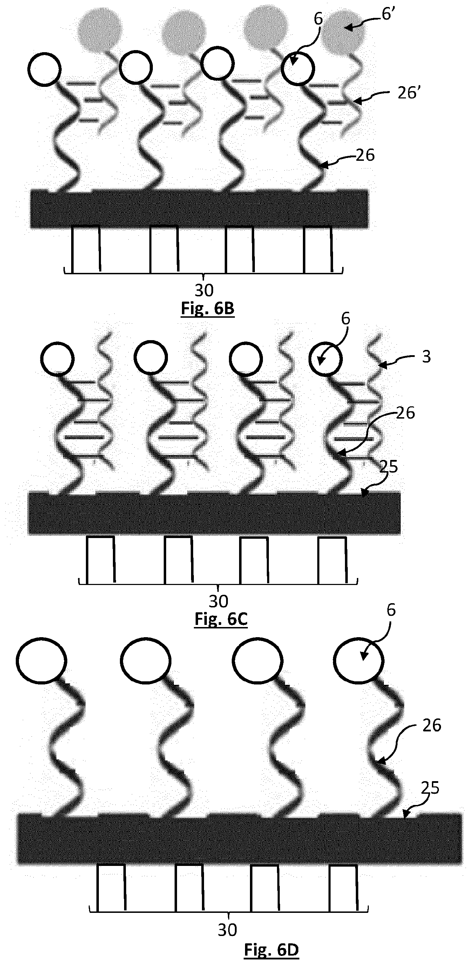

Selon une autre possibilité, représentée sur les figures 6B et 6C, la sonde biologique de détection 26 est marquée à l'aide d'un marqueur fluorescent 6. Elle est également liée à une structure complémentaire 26' portant un désactivateur de fluorescence 6'. Le désactivateur de fluorescence est usuellement désigné par le terme « quencher », le terme « quench » signifiant éteindre. En présence d'un désactiveur (cf. figure 6B), un transfert d'énergie est effectué entre le fluorophore et le désactivateur. La sonde biologique de détection 26 présente plus d'affinité à l'égard de la biomolécule d'intérêt qu'à l'égard de la structure complémentaire 26'. En l'absence de biomolécule d'intérêt 3, ou d'une quantité suffisante de la biomolécule d'intérêt, dans l'échantillon 2, l'action du quencher induit une émission négligeable d'une fluorescence par la sonde biologique de détection 26. En présence d'une biomolécule d'intérêt 3 adsorbée sur la sonde de détection 26 (cf. figure 6C), cette dernière tend à se fixer sur la sonde biologique de détection 26, à la place de la structure complémentaire 26'. La disparition du quencher 6' entraîne une émission d'une lumière de fluorescence par le marqueur fluorescent 6, traduisant la capture de la biomolécule d'intérêt 3.

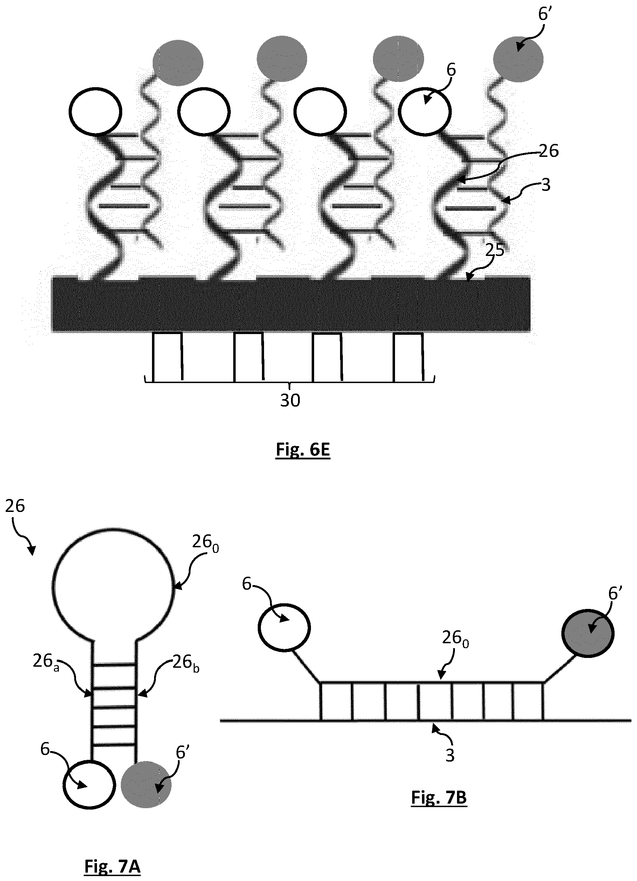

Selon une autre possibilité, représentée sur les figures 6D et 6E, chaque sonde de détection biologique 26 est liée à un marqueur fluorescent 6 et la biomolécule d'intérêt 3 est liée à un quencher. En l'absence de biomolécule d'intérêt 3 couplée à la sonde de détection 26, une lumière de fluorescence 8 est émise. Cf. figure 6D. En présence d'une biomolécule d'intérêt 3 adsorbée sur la sonde de détection 26, le quencher bloque l'émission de la lumière de fluorescence. Ainsi, la capture d'une biomolécule d'intérêt se traduit par une diminution de la lumière de fluorescence. Cf. figure 6E.

Les figures 7A et 7B schématisent une structure préférentielle de sonde de détection biologique 26. Un telle structure est usuellement désignée balise moléculaire, ou « molecular beacon ». Ce type de sonde est destiné à une capture d'une séquence monobrin de nucléotides. Sur la figure 7A, la sonde de détection 26 comporte deux bras complémentaires 26ₐ, 26_{b} s'étendant parallèlement l'une à l'autre, à partir d'une boucle 26₀. Un marqueur fluorescent est greffé par liaison covalente à l'extrémité d'un bras 26ₐ tandis qu'un quencher est greffé par liaison covalente à l'extrémité sur le bras complémentaire 26_{b}. La boucle 26₀ est constituée d'une séquence complémentaire de la biomolécule d'intérêt 3. En l'absence de biomolécule d'intérêt 3, la sonde de détection reste fermée, en forme d'épingle à cheveux. La présence du quencher 6' évite ou limite l'émission d'une lumière de fluorescence par le marqueur fluorescent 6. L'adsorption d'une biomolécule d'intérêt 3 au niveau de la boucle 26₀ entraîne une ouverture de la balise moléculaire, comme représenté sur la figure 7B. L'éloignement du marqueur fluorescent 6 vis-à-vis quencher 6' empêche le transfert d'énergie conduisant à l'extinction de l'émission fluorescente. Ainsi, la lumière de fluorescence émise par le marqueur fluorescent 6 est émise et peut être détectée par un nanofil. Avec ce type de sonde de détection, la capture d'une biomolécule d'intérêt se traduit par une augmentation de la lumière de fluorescence.

Le fonctionnement d'une balise moléculaire telle que schématisée sur les figures 7A et 7B suppose que l'affinité de la boucle 26₀ vis-à-vis de la biomolécule d'intérêt soit suffisamment élevée pour que les deux bras complémentaires 26ₐ, 26_{b} puissent être découplés.

Ce type de balise moléculaire peut être greffé sur la surface de fonctionnalisation 25 en formant des liaisons carboxyles sur la surface de détection. Lorsque la couche de contact est formée de PMMA, les fonctions carboxyles peuvent être formées en surface par un traitement plasma oxygène. Les balises peuvent comporter une fonction amine. La fonctionnalisation de la surface de détection est obtenue par la formation de liaisons covalentes avec la fonction carboxyle à la surface de fonctionnalisation 25.

### Structure des nanofils

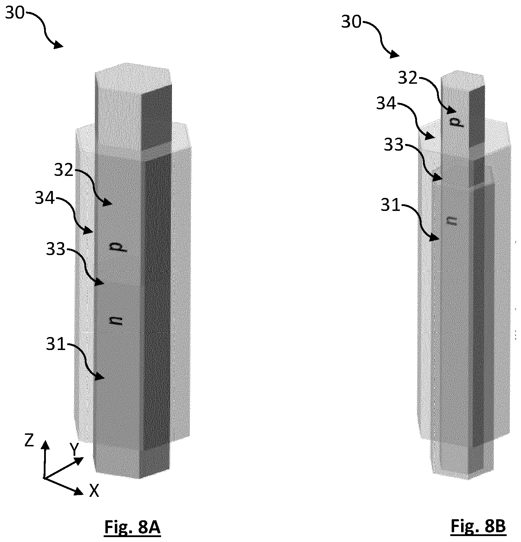

Les figures 8A et 8B représentent d'autres structures de nanofils pouvant être mises en oeuvre dans un dispositif selon l'invention. La figure 8A représente un nanofil similaire aux nanofils précédemment décrits. La jonction 33 est disposée axialement, en s'étendant entre deux parties 31, 32, de dopage différents, espacées l'une de l'autre selon l'axe transversal Z. Dans l'exemple de la figure 8A, une gaine de passivation 34 contourne le nanofil.

Dans l'exemple de la figure 8B, la jonction 33 s'étend radialement entre deux zones de dopage différents. Ainsi, la jonction 33 s'étend autour de l'axe transversal Z, parallèlement à ce dernier. La première partie 31 et la deuxième partie 32 sont séparées radialement, la séparation entre les deux parties correspondant à un rayon de séparation. La première partie s'étend entre l'axe du nanofil et la jonction 33, tandis que la deuxième partie s'étend autour de la jonction 33.

Une structure axiale, est considérée comme avantageuse car elle favorise une incorporation de puits quantiques ou de points quantiques à l'intérieur des nanofils afin d'ajuster le spectre d'absorption.

Une structure radiale permet de disposer d'une jonction 33 s'étendant selon une hauteur importante selon l'axe Z, ce qui permet d'augmenter la sensibilité de détection. De façon optionnelle, la structure radiale représentée sur la figure 8B comporte une gaine annulaire 34 telle que décrite en lien avec la figure 8A.

Les structures de nanofils décrites en lien avec la figure 8A peuvent être réalisées selon une approche descendante, par exemple par gravure, comme décrit en lien avec les figures 4A et 4B, en adaptant la constitution les couches déposées sur le substrat 10. Les structures de nanofils décrites en lien avec la figure 8B peuvent être réalisées par croissance de nanofils.

### Applications

Le dispositif précédemment décrit peut être utilisé pour la détection d'hybridation entre deux fragments complémentaires d'ADN, l'un formant la sonde de détection, l'autre formant la biomolécule cible. Les applications peuvent concerner l'amplification par PCR (Polymerase Chain Reaction - Réaction en Chaîne Polymérase). On estime que le dispositif permet une détection plus rapide d'une amplification : en effet, la sensibilité du dispositif permet de distinguer entre une copie et deux copies de la même biomolécule fluorescente, soit dès la première séquence d'amplification par duplication, par opposition aux dispositifs à l'état de l'art qui nécessitent au moins 5 cycles d'amplification (2⁵ copies). Le dispositif peut être utilisé dans le séquençage, par exemple le séquençage haut débit d'ADN. On tire profit de la sensibilité des nanofils, permettant de diminuer le nombre de copies d'une séquence de nucléotides examinée. De plus, comme préalablement décrit, le dispositif permet de disposer de plusieurs grappes de nanofils adressant respectivement différentes séquences de nucléotides. Cela permet d'effectuer plusieurs détections en parallèle. Chaque grappe de nanofils est alors associée à un nanopuits 27 formé dans la couche d'interface 23. Chaque nanopuits est ensemencé par un seul brin d'ADN issu d'une librairie précédemment préparée suivant des techniques connues. Chaque brin est simultanément et compétitivement amplifié (par exemple par amplification par pont) afin de coloniser toute la surface du nanopuits avec des brins monoclonaux, formant ainsi des grappes de brins d'ADN monoclonaux alignés avec les grappes de nanofils. Les brins d'ADN sont ensuite hybridés base par base avec des bases complémentaires suivant un protocole dit « one-channel » (un canal) comportant deux cycles :
- Lors du premier cycle, par exemple, les bases A (adénine) et T (thymine) du brin à séquencer sont hybridées à une base complémentaire sur laquelle est greffé un fluorophore émettant dans la longueur d'onde détectée par les nanofils. La fluorescence est alors enregistrée, correspondant aux bases A ou T.
- Le second cycle permet de cliver le fluorophore ayant permis d'identifier les bases A, de le conserver sur les bases T, et de greffer un fluorophore les bases C (cytosine). La fluorescence est à nouveau enregistrée, correspondant cette fois aux bases T ou C.
- Par combinaison des deux enregistrements, on en déduit la base hybridée pour chaque cluster monoclonal : base A si un signal fluorescent a été enregistré lors du premier cycle mais pas lors du second ; base T si un signal fluorescent a été enregistré lors des deux cycles ; base C si un signal fluorescent a été enregistré lors du second cycle mais pas lors du premier ; base G (guanine) si aucun fluorescence n'a été détectée lors des premier et second cycles.

Cette séquence est répétée autant de fois qu'il y a de bases à séquencer.

Lors de l'utilisation du dispositif pour la détection d'hybridation d'ADN, on préfèrera marquer les sondes de détection plutôt que les cibles. En cela, le recours à des balises moléculaires, ne nécessitant pas de marquage des cibles, est préféré.

Le dispositif peut également être appliqué à une détection d'un greffage de type anticorps - antigène.

Le dispositif tire profit d'un temps de réponse rapide, typiquement de l'ordre de la ns. De plus, l'utilisation de différentes sondes biologiques, adressant respectivement différentes biomolécules cibles, isolées les unes des autres sur la surface de détection, permet d'adresser simultanément différentes biomolécules cibles. On peut obtenir, pour chaque échantillon, une réponse dite digitale : la présence et l'absence des différentes biomolécules cibles est obtenue simultanément, la réponse étant 1 en cas de présence et 0 en cas d'absence de quantité détectable.

On observe que le dispositif ne nécessite pas le recours à des composants optiques volumineux. De plus, la réponse du dispositif est stable, et peu sensibles aux variations environnementales : pH de l'échantillon, température, présence de molécules ou d'ions différents de la biomolécule d'intérêt. Cela est dû au fait que les nanofils ne sont pas au contact de l'échantillon, mais isolés, physiquement et électriquement, de ce dernier par la couche d'interface 23.

Enfin, le dispositif permet d'effectuer des mesures sans étalonnage systématique. Par exemple lors de tests ELISA (enzyme-linked immunosorbent assay - technique d'immunoabsorption par enzyme liée), il est typiquement nécessaire d'étalonner les systèmes existants à chaque mesure sur une gamme de dilution, afin de s'affranchir de l'instabilité globale du système (composants optiques...). Notre système, par sa stabilité, permet de n'être étalonné qu'une fois pour toutes les mesures effectuées dans un délai garantissant la stabilité des réactifs : ces derniers deviennent le seul élément limitant nécessitant une recalibration périodique. Enfin, le dispositif étant basé sur des nanophotodétecteurs, il permet d'obtenir une plateforme d'analyse compacte. Le dispositif peut être obtenu en mettant en oeuvre un procédé de fabrication collective, ce qui permet d'abaisser le coût.

## Revendications

1. Dispositif (1) de détection d'au moins une biomolécule d'intérêt (3) dans un échantillon (2), le dispositif comportant :
- un substrat (10), comportant au moins une première électrode (11_{c});
- une structure multicouches (20), comportant au moins une deuxième électrode (21_{c});
- des nanofils (30), s'étendant entre la première électrode (11_{c}) et la deuxième électrode (21_{c}), parallèlement à un axe transversal (Z) ;
- une couche d'encapsulation (15) s'étendant autour des nanofils, entre le substrat et la structure multicouches (20), la couche d'encapsulation étant formée d'un matériau isolant ;
- la structure multicouches comportant :
• une couche conductrice (22), formant chaque deuxième électrode;
• une couche d'interface (23), électriquement isolante, recouvrant chaque deuxième électrode (21_{c}), chaque deuxième électrode étant interposée entre la couche d'interface et un nanofil, la couche d'interface étant délimitée par une surface de fonctionnalisation (25), la couche d'interface étant configurée pour être disposée entre l'échantillon et la deuxième électrode, de telle sorte que la surface de fonctionnalisation forme une interface entre le dispositif et l'échantillon ;
- la structure multicouche étant telle que
• la surface de fonctionnalisation est configurée pour capturer sélectivement la biomolécule d'intérêt;
• la deuxième électrode et la couche d'interface sont transparentes dans une bande spectrale de détection ;
le dispositif étant tel que :
- chaque nanofil (30) comporte une jonction (33) de type homojonction, ou une hétérojonction, ou une jonction Schottky entre la première électrode et la deuxième électrode ;
- la première électrode et la deuxième électrode sont configurées pour être raccordées à un circuit de détection (40);
- de telle sorte que chaque nanofil forme un nanophotodétecteur d'une lumière émise au niveau de la surface de fonctionnalisation, la lumière détectée par chaque nanofil induisant un signal électrique de détection dans le circuit de détection.

2. Dispositif selon la revendication 1, dans lequel chaque nanofil comporte :
- une homojonction de type pn ;
- ou une hétérojonction ;
- ou une jonction Schottky de type p-métal ou n-métal.

3. Dispositif selon l'une quelconque des revendications précédentes, dans lequel au moins un nanofil comporte une première partie (31) et une deuxième partie (32), séparées par la jonction, la première partie étant formée d'un semi-conducteur dopé n, la deuxième partie étant formée d'un semi-conducteur dopé p, la jonction formant une homojonction ou une hétérojonction.

4. Dispositif selon la revendication 2, dans lequel au moins un nanofil comporte une première partie (31) et une deuxième partie (32), séparées par la jonction, la première partie étant formée d'un semi-conducteur, la deuxième partie, adjacente d'une électrode, étant formée d'un métal, la jonction formant une jonction Schottky.

5. Dispositif selon l'une quelconque des revendications 3 ou 4, dans lequel la première partie et la deuxième partie s'étendent, selon l'axe transversal, de part et d'autre de la jonction.

6. Dispositif selon l'une quelconque des revendications 3 ou 4, dans lequel la deuxième partie entoure la première partie, autour de l'axe transversal.

7. Dispositif selon l'une quelconque des revendications précédentes, dans lequel au moins un nanofil comporte un puits quantique ou un point quantique au niveau de la jonction.

8. Dispositif selon l'une quelconque des revendications précédentes, dans lequel la surface de fonctionnalisation (25) est segmentée en différentes surfaces élémentaires, chaque surface élémentaire formant un site de capture de la biomolécule d'intérêt.

9. Dispositif selon la revendication 8 dans lequel :
- la couche d'interface comporte deux sous-couches (23₁, 23₂), empilées l'une sur l'autre, formant une sous-couche inférieure et une sous-couche supérieure, la sous-couche inférieure étant interposée entre la couche conductrice (22) et la sous-couche supérieure ;
- la sous-couche supérieure comporte des puits, débouchant dans la sous-couche inférieure, chaque puits étant disposé face à un nanofil, chaque puits formant une partie de la surface de fonctionnalisation ;
- la surface de fonctionnalisation est segmentée au niveau de chaque puits, de façon que chaque puits forme un site de capture de la biomolécule d'intérêt.

10. Dispositif selon l'une quelconque des revendications précédentes, comportant plusieurs nanofils, s'étendant entre une même première électrode et une même deuxième électrode, les nanofils formant une grappe de nanofils.

11. Dispositif selon la revendication 10, comportant plusieurs grappes de nanofils (35), distantes les unes des autres, de telle sorte qu'un nanofil d'une grappe est plus proche d'un autre nanofil de ladite grappe que d'un autre nanofil d'une autre grappe, les nanofils d'une même grappe s'étendant entre une même première électrode et une même deuxième électrode.

12. Dispositif selon l'une quelconque des revendications précédentes, comportant plusieurs nanofils, le dispositif étant tel que :
- plusieurs premières électrodes sont formées sur le substrat, et plusieurs deuxièmes électrodes sont formées sur la structure multicouche, chaque nanofil s'étendant entre une première électrode et une deuxième électrode ;
- chaque première électrode est reliée à une première unité d'adressage (40_{X}), configurée pour sélectionner au moins une première électrode ;
- chaque deuxième électrode est reliée à une deuxième unité d'adressage (40_{Y}), configurée pour sélectionner au moins une deuxième électrode ;
- de telle sorte que le circuit de détection (40) détecte un signal de détection induit par chaque nanofil s'étendant entre la première électrode et la deuxième électrode sélectionnées.

13. Dispositif selon l'une quelconque des revendications précédentes, dans lequel :
- la surface de fonctionnalisation est fonctionnalisée par une sonde biologique de détection, la sonde biologique de détection étant configurée pour capturer sélectivement une biomolécule d'intérêt ;
- de telle sorte qu'une capture de la biomolécule d'intérêt entraîne une variation de la lumière de fluorescence détectée par au moins un nanofil lorsque la surface de fonctionnalisation est éclairée par une lumière d'excitation d'un marqueur fluorescent greffé sur la biomolécule d'intérêt ou sur la sonde biologique de détection.

14. Dispositif selon la revendication 13, dans lequel :
- la surface de fonctionnalisation est segmentée en plusieurs portions (25₁, 25₂);
- chaque portion de la surface de fonctionnalisation s'étend face à au moins un nanofil ;
- différentes portions de la surface de fonctionnalisation (25₁, 25₂)sont respectivement fonctionnalisées par des sondes biologiques de détection (26₁, 26₂) configurées pour capturer sélectivement différentes biomolécules d'intérêt.

15. Procédé de détection d'au moins une biomolécule d'intérêt (3) à l'aide d'un dispositif selon l'une quelconque des revendications précédentes, la surface de fonctionnalisation étant préalablement fonctionnalisée avec une sonde biologique de détection configurée pour capturer sélectivement la biomolécule d'intérêt, le procédé comportant :
a) disposition d'un échantillon, susceptible de contenir la biomolécule d'intérêt, au contact de la surface de fonctionnalisation ;
b) connexion du circuit de détection aux bornes d'une première électrode et d'une deuxième électrode ;
c) exposition de la surface de fonctionnalisation à une lumière d'excitation, dans une bande spectrale d'excitation d'un marqueur fluorescent, le marqueur fluorescent étant greffé sur la sonde biologique de détection ou sur la biomolécule d'intérêt, le marqueur fluorescent étant susceptible d'émettre une lumière de fluorescence, dans la bande spectrale de détection, lorsqu'il est illuminé par la lumière d'excitation ;
d) en fonction du signal de détection, détection d'une variation d'une lumière de fluorescence détectée par au moins un nanofil, la variation traduisant une capture de la biomolécule d'intérêt par une sonde biologique de détection.

16. Procédé selon la revendication 15, dans lequel :
- le dispositif est un dispositif selon la revendication 12 ;
- les étapes b) à d) sont réitérées, en modifiant, entre deux itérations successives, la première électrode sélectionnée ou la deuxième électrode sélectionnée.

## Patentansprüche

1. Vorrichtung (1) zur Detektion mindestens eines Biomoleküls von Interesse (3) in einer Probe (2), wobei die Vorrichtung Folgendes umfasst:
- ein Substrat (10), das mindestens eine erste Elektrode (11_{c}) umfasst;
- eine mehrschichtige Struktur (20), die mindestens eine zweite Elektrode (21_{c}) umfasst;
- Nanodrähte (30), die sich parallel zu einer Querachse (Z) zwischen der ersten Elektrode (11_{c}) und der zweiten Elektrode (21_{c}) erstrecken;
- eine Verkapselungsschicht (15), die sich zwischen dem Substrat und der mehrschichtigen Struktur (20) um die Nanodrähte erstreckt, wobei die Verkapselungsschicht aus einem Isoliermaterial gebildet ist;
- wobei die mehrschichtige Struktur Folgendes umfasst:
· eine leitende Schicht (22), die jede zweite Elektrode bildet;
· eine elektrisch isolierende Grenzflächenschicht (23), die jede zweite Elektrode (21_{c}) bedeckt, wobei jede zweite Elektrode zwischen der Grenzflächenschicht und einem Nanodraht eingefügt ist, wobei die Grenzflächenschicht durch eine Funktionalisierungsoberfläche (25) begrenzt wird, wobei die Grenzflächenschicht dazu konfiguriert ist, zwischen der Probe und der zweiten Elektrode angeordnet zu sein, sodass die Funktionalisierungsoberfläche eine Grenzfläche zwischen der Vorrichtung und der Probe bildet;
- wobei die mehrschichtige Struktur derart ist, dass
• die Funktionalisierungsoberfläche dazu konfiguriert ist, das Biomolekül von Interesse selektiv einzufangen;
• die zweite Elektrode und die Grenzflächenschicht in einem Detektionspektralbereich transparent sind;
wobei die Vorrichtung derart ist, dass:
- jeder Nanodraht (30) einen Übergang (33) vom Typ Homoübergang oder einen Heteroübergang oder einen Schottky-Übergang zwischen der ersten Elektrode und der zweiten Elektrode umfasst;
- die erste Elektrode und die zweite Elektrode dazu konfiguriert sind, mit einer Detektionsschaltung (40) verbunden zu sein;
- sodass jeder Nanodraht für Licht, das im Bereich der Funktionalisierungsoberfläche emittiert wird, einen Nanofotodetektor bildet, wobei das durch jeden Nanodraht detektierte Licht in der Detektionsschaltung ein elektrisches Detektionssignal induziert.

2. Vorrichtung nach Anspruch 1, wobei jeder Nanodraht Folgendes umfasst:
- einen Homoübergang vom Typ pn;
- oder einen Heteroübergang;
- oder einen Schottky-Übergang vom Typ p-Metall oder n-Metall.

3. Vorrichtung nach einem beliebigen der vorhergehenden Ansprüche, wobei mindestens ein Nanodraht einen ersten Teil (31) und einen zweiten Teil (32) umfasst, die durch den Übergang getrennt sind, wobei der erste Teil aus einem n-dotierten Halbleiter gebildet ist, wobei der zweite Teil aus einem p-dotierten Halbleiter gebildet ist, wobei der Übergang einen Homoübergang oder einen Heteroübergang bildet.

4. Vorrichtung nach Anspruch 2, wobei mindestens ein Nanodraht einen ersten Teil (31) und einen zweiten Teil (32) umfasst, die durch den Übergang getrennt sind, wobei der erste Teil aus einem Halbleiter gebildet ist, wobei der zweite Teil, angrenzend an eine Elektrode, aus einem Metall gebildet ist, wobei der Übergang einen Schottky-Übergang bildet.

5. Vorrichtung nach einem beliebigen der Ansprüche 3 oder 4, wobei sich der erste Teil und der zweite Teil gemäß der Querachse zu beiden Seiten des Übergangs erstrecken.

6. Vorrichtung nach einem beliebigen der Ansprüche 3 oder 4, wobei der zweite Teil den ersten Teil um die Querachse herum umgibt.

7. Vorrichtung nach einem beliebigen der vorhergehenden Ansprüche, wobei mindestens ein Nanodraht einen Quantentopf oder einen Quantenpunkt im Bereich des Übergangs umfasst.

8. Vorrichtung nach einem beliebigen der vorhergehenden Ansprüche, wobei die Funktionalisierungsoberfläche (25) in unterschiedliche Elementaroberflächen unterteilt ist, wobei jede Elementaroberfläche einen Einfangbereich für das Biomolekül von Interesse bildet.

9. Vorrichtung nach Anspruch 8, wobei:
- die Grenzflächenschicht zwei übereinander gestapelte Teilschichten (23₁, 23₂) umfasst, die eine untere Teilschicht und eine obere Teilschicht bilden, wobei die untere Teilschicht zwischen der leitenden Schicht (22) und der oberen Teilschicht eingefügt ist;
- die obere Teilschicht Wannen umfasst, die in der unteren Teilschicht münden, wobei jede Wanne gegenüber einem Nanodraht angeordnet ist, wobei jede Wanne einen Teil der Funktionalisierungsoberfläche bildet;
- die Funktionalisierungsoberfläche im Bereich jeder Wanne unterteilt ist, sodass jede Wanne einen Einfangbereich für das Biomolekül von Interesse bildet.

10. Vorrichtung nach einem beliebigen der vorhergehenden Ansprüche, die mehrere Nanodrähte, die sich zwischen einer gleichen ersten Elektrode und einer gleichen zweiten Elektrode erstrecken, umfasst, wobei die Nanodrähte einen Nanodraht-Cluster bilden.

11. Vorrichtung nach Anspruch 10, die mehrere Nanodraht-Cluster (35) umfasst, die voneinander beabstandet sind, sodass sich ein Nanodraht eines Clusters näher an einem anderen Nanodraht des Clusters als an einem anderen Nanodraht eines anderen Clusters befindet, wobei sich die Nanodrähte eines gleichen Clusters zwischen einer gleichen ersten Elektrode und einer gleichen zweiten Elektrode erstrecken.

12. Vorrichtung nach einem beliebigen der vorhergehenden Ansprüche, die mehrere Nanodrähte umfasst, wobei die Vorrichtung derart ist, dass:
- mehrere erste Elektroden auf dem Substrat gebildet sind und mehrere zweite Elektroden auf der mehrschichtigen Struktur gebildet sind, wobei sich jeder Nanodraht zwischen einer ersten Elektrode und einer zweiten Elektrode erstreckt;
- jede erste Elektrode mit einer ersten Adressiereinheit (40_{X}) verbunden ist, die dazu konfiguriert ist, mindestens eine erste Elektrode auszuwählen;
- jede zweite Elektrode mit einer zweiten Adressiereinheit (40_{Y}) verbunden ist, die dazu konfiguriert ist, mindestens eine zweite Elektrode auszuwählen;
- sodass die Detektionsschaltung (40) ein Detektionssignal detektiert, das durch jeden Nanodraht, der sich zwischen der ausgewählten ersten Elektrode und der ausgewählten zweiten Elektrode erstreckt, induziert wird.

13. Vorrichtung nach einem beliebigen der vorhergehenden Ansprüche, wobei:
- die Funktionalisierungsoberfläche durch eine biologische Detektionssonde funktionalisiert wird, wobei die biologische Detektionssonde dazu konfiguriert ist, ein Biomolekül von Interesse selektiv einzufangen;
- sodass ein Einfangen des Biomoleküls von Interesse eine Änderung des Fluoreszenzlichts, die durch mindestens einen Nanodraht detektiert wird, bewirkt, wenn die Funktionalisierungsoberfläche mit einem Licht zur Anregung eines Fluoreszenzmarkers, der an das Biomolekül von Interesse oder an die biologische Detektionssonde angelagert ist, beleuchtet wird.

14. Vorrichtung nach Anspruch 13, wobei:
- die Funktionalisierungsoberfläche in mehrere Abschnitte (25₁, 25₂)unterteilt ist;
- sich jeder Abschnitt der Funktionalisierungsoberfläche gegenüber mindestens einem Nanodraht erstreckt;
- unterschiedliche Abschnitte der Funktionalisierungsoberfläche (25₁, 25₂)jeweils durch biologische Detektionssonden (26₁, 26₂) funktionalisiert werden, die dazu konfiguriert sind, unterschiedliche Biomoleküle von Interesse selektiv einzufangen.

15. Verfahren zur Detektion mindestens eines Biomoleküls von Interesse (3) mit Hilfe einer Vorrichtung nach einem beliebigen der vorhergehenden Ansprüche, wobei die Funktionalisierungsoberfläche vorab mit einer biologischen Detektionssonde funktionalisiert wird, die dazu konfiguriert ist, das Biomolekül von Interesse selektiv einzufangen, wobei das Verfahren Folgendes umfasst:
a) Anordnen einer Probe, die das Biomolekül von Interesse enthalten kann, in Kontakt mit der Funktionalisierungsoberfläche;
b) Verbinden der Detektionsschaltung mit den Anschlüssen einer ersten Elektrode und einer zweiten Elektrode;
c) Aussetzen der Funktionalisierungsoberfläche gegenüber einem Anregungslicht in einem Anregungsspektralbereich eines Fluoreszenzmarkers, wobei der Fluoreszenzmarker an die biologische Detektionssonde oder an das Biomolekül von Interesse angelagert ist, wobei der Fluoreszenzmarker ein Fluoreszenzlicht in dem Detektionsspektralbereich emittieren kann, wenn er mit dem Anregungslicht beleuchtet wird;
d) in Abhängigkeit von dem Detektionssignal, Detektieren einer Änderung eines Fluoreszenzlichts, die durch mindestens einen Nanodraht detektiert wird, wobei die Änderung ein Einfangen des Biomoleküls von Interesse durch eine biologische Detektionssonde ausdrückt.

16. Vorrichtung nach Anspruch 15, wobei:
- die Vorrichtung eine Vorrichtung nach Anspruch 12 ist;
- die Schritte b) und d) wiederholt werden, wobei zwischen zwei aufeinanderfolgenden Wiederholungen die erste ausgewählte Elektrode oder die zweite ausgewählte Elektrode geändert wird.

## Claims

1. Device (1) for detecting at least one biomolecule of interest (3) in a sample (2), the device comprising:
- a substrate (10), comprising at least a first electrode (11_{c});
- a multilayer structure (20), comprising at least a second electrode (21_{c});
- nanowires (30), extending between the first electrode (11_{c}) and the second electrode (21_{c}), parallel to a transverse axis;
- an encapsulation layer (15) extending around the nanowires, between the substrate and the multilayer structure (20), the encapsulation layer being formed from an insulating material;
- the multilayer structure comprising:
• a conductive layer (22), forming each second electrode;
• an electrically insulating interface layer (23) covering each second electrode (21_{c}), each second electrode being interposed between the interface layer and a nanowire, the interface layer being bounded by a functionalization surface (25), the interface layer being configured to be placed between the sample and the second electrode, such that the functionalization surface forms an interface between the device and the sample;
- the multilayer structure being such that
• the functionalization surface is configured to selectively capture the biomolecule of interest;
• the second electrode and the interface layer are transparent in a detection spectral band;
the device being such that:
- each nanowire (30) comprises a homojunction, or heterojunction, or Schottky junction (33) between the first electrode and the second electrode;
- the first electrode and the second electrode are configured to be connected to a detection circuit (40);
- such that each nanowire forms a nanophotodetector of light emitted from the functionalization surface, the light detected by each nanowire inducing a detection electrical signal in the detection circuit.

2. Device according to Claim 1, wherein each nanowire comprises:
- a p-n homojunction;
- a heterojunction;
- a p-metal or n-metal Schottky junction.

3. Device according any one of the preceding claims, wherein at least one nanowire comprises a first portion (31) and a second portion (32), which are separated by the junction, the first portion being formed from an n-doped semiconductor, the second portion being formed from a p-doped semiconductor, the junction forming a homojunction or a heterojunction.

4. Device according to Claim 2, wherein at least one nanowire comprises a first portion (31) and a second portion (32), which are separated by the junction, the first portion being formed from a semiconductor, the second portion, adjacent an electrode, being formed from a metal, the junction forming a Schottky junction.

5. Device according to any one of claims 3 or 4, wherein the first portion and the second portion lie, along the transverse axis, on either side of the junction.

6. Device according to any one of claims 3 or 4, wherein the second portion encircles the first portion, around the transverse axis.

7. Device according to any one of the preceding claims, wherein at least one nanowire comprises a quantum well, or a quantum dot at the junction.

8. Device according to any one of the preceding claims, wherein the functionalization surface (25) is segmented into various elementary surfaces, each elementary surface forming a site of capture of the biomolecule of interest.

9. Device according to Claim 8, wherein:
- the interface layer comprises two sublayers (23₁, 23₂), stacked on each other, forming a lower sublayer and an upper sublayer, the lower sublayer being interposed between the conductive layer (22) and the upper sublayer;
- the upper sublayer comprises wells, opening into the lower sublayer, each well being placed facing one nanowire, each well forming one portion of the functionalization surface;
- the functionalization surface is segmented level with each well, so that each well forms a site of capture of the biomolecule of interest.

10. Device according to any one of the preceding claims, comprising a plurality of nanowires, extending between the same first electrode and the same second electrode, the nanowires forming a bunch of nanowires.

11. Device according to Claim 10, comprising a plurality of bunches of nanowires (35), which are separated from one another, such that any nanowire of a bunch is closer to another nanowire of said bunch than to another nanowire of another bunch, the nanowires of a given bunch extending between the same first electrode and the same second electrode.

12. Device according to any one of the preceding claims, comprising a plurality of nanowires, the device being such that:
- a plurality of first electrodes are formed on the substrate, and a plurality of second electrodes are formed on the multilayer structure, each nanowire extending between a first electrode and a second electrode;
- each first electrode is connected to a first addressing unit (40x), configured to select at least one first electrode;
- each second electrode is connected to a second addressing unit (40_{Y}), configured to select at least one second electrode;
- such that the detection circuit (40) detects a detection signal induced by each nanowire extending between the selected first electrode and the selected second electrode.

13. Device according to any one of the preceding claims, wherein:
- the functionalization surface is functionalized by a biological detection probe, the biological detection probe being configured to selectively capture a biomolecule of interest;
- such that capture of the biomolecule of interest leads to a variation in the fluorescence light detected by at least one nanowire when the functionalization surface is illuminated with excitation light to excite a fluorescent label grafted to the biomolecule of interest or to the biological detection probe..

14. Device according to Claim 13, wherein:
- the functionalization surface is segmented into a plurality of segments;
- each segment of the functionalization surface lies facing at least one nanowire;
- various segments of the functionalization surface are functionalized by biological detection probes configured to selectively capture various biomolecules of interest, respectively.

15. Method for detecting at least one biomolecule of interest using a device according to Claim 1, the functionalization surface being functionalized beforehand with a biological detection probe configured to selectively capture the biomolecule of interest, the method comprising:
a) placing a sample, liable to contain the biomolecule of interest, in contact with the functionalization surface;
b) connecting the detection circuit to the terminals of a first electrode and of a second electrode;
c) exposing the functionalization surface to excitation light, in an excitation spectral band of a fluorescent label, the fluorescent label being grafted to the biological detection probe or to the biomolecule of interest, the fluorescent label being capable of emitting fluorescent light, in the detection spectral band, when it is illuminated by the excitation light;
d) depending on the detection signal, detecting a variation in fluorescence light detected by at least one nanowire, the variation indicating capture of the biomolecule of interest by a biological detection probe.

16. Method according to Claim 15, wherein:
- the device is a device according to Claim 12;
- steps b) to d) are reiterated, modifying, between two successive iterations, the selected first electrode or the selected second electrode.
